(19) Europäisches Patentamt
European Patent Office
Office européen des brevets

(11) Veröffentlichungsnummer: **0 505 819 A1**

(12) **EUROPÄISCHE PATENTANMELDUNG**

(21) Anmeldenummer: **92104042.4**

(51) Int. Cl.5: **C07D 249/12**, A01N 47/38

(22) Anmeldetag: **10.03.92**

(30) Priorität: **23.03.91 DE 4109671**

(43) Veröffentlichungstag der Anmeldung:
**30.09.92 Patentblatt 92/40**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Müller, Klaus-Helmut, Dr.**
**Bockhackstrasse 55**
**W-4000 Düsseldorf 13(DE)**
Erfinder: **Findeisen, Kurt, Prof. Dr.**
**Dünfelder Strasse 28**
**W-5090 Leverkusen 1(DE)**
Erfinder: **Kuhnt, Dietmar, Dr.**
**Körnerstrasse 5**
**W-5090 Leverkusen 1(DE)**
Erfinder: **König, Klaus, Dr.**
**Zum Hahnenberg 40**
**W-5068 Odenthal(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Substituierte Triazolinone.**

(57) Die Erfindung betrifft neue substituierte Triazolinone, der Formel (I),

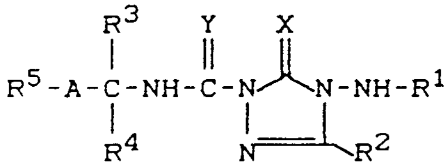

(I)

in welcher
A für Alkandiyl, Alkendiyl oder Alkindiyl steht,
X und Y jeweils für O oder S stehen und die Reste $R^1$ bis $R^5$ die in der Beschreibung genannten Bedeutungen haben,
Verfahren zu ihrer Herstellung und ihre Verwendung als Herbizide.

EP 0 505 819 A1

Die Erfindung betrifft neue substituierte Triazolinone, mehrere Verfahren zu ihrer Herstellung und ihre Verwendung als Herbizide.

Es ist bekannt, daß bestimmte substituierte Triazolinone wie beispielsweise 2-(Phenylaminocarbonyl)-4-amino-5-methyl-2,4-dihydro-3H-1,2,4-triazol-3-on (vgl. EP-A 294 666; vgl. auch EP-A 399 294) herbizide Eigenschaften besitzen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiterhin sind bestimmte substituierte Triazolinone, wie beispielsweise das 2-(N,N-Dimethylaminocarbonyl)-4-amino-5-phenyl-2,4-dihydro-3H-1,2,4-triazol-3-on bekannt (vergl. J.Heterocycl. Chem. 17, 1691-1696 [1980]; Org. Mass. Spectrom. 14, 369-378 [1979]).

Über eine Wirksamkeit dieser vorbekannten Triazolinone als Herbizide ist bisher nichts bekannt.

Es wurden neue substituierte Triazolinone der allgemeinen Formel (I),

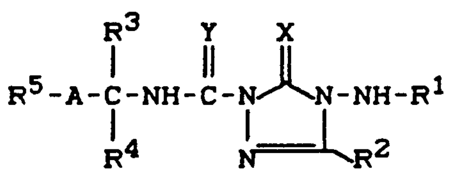

(I)

in welcher

| | |
|---|---|
| A | für Alkandiyl, Alkendiyl oder Alkindiyl steht, |
| $R^1$ | für Alkyl, Alkenyl, Alkinyl, Halogenalkyl, Halogenalkenyl, Halogenalkinyl, Cyanalkyl, Alkoxyalkyl, Cycloalkyl, Halogencycloalkyl, Alkylcycloalkyl oder Cycloalkylalkyl steht, |
| $R^2$ | für Alkyl, Halogenalkyl, Alkoxyalkyl oder Cycloalkyl steht, |
| $R^3$ | für Wasserstoff oder Alkyl steht, |
| $R^4$ | für Alkyl steht, |
| $R^5$ | für jeweils gegebenenfalls substituiertes Aryl oder Cycloalkyl steht und |
| X und Y | unabhängig voneinander für Sauerstoff oder Schwefel stehen, |

gefunden, wobei folgende Verbindungen (bekannt aus EP-A 399 294) ausgenommen sind: 2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

Die Verbindungen der Formel (I) können gegebenenfalls in Abhängigkeit von der Art der Substituenten $R^1$ bis $R^5$ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I) erhält, wenn man

(a) Triazolinone der allgemeinen Formel (II)

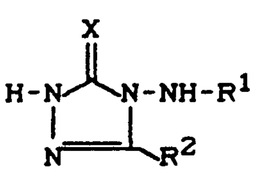

(II)

in welcher

$R^1$, $R^2$ und X die oben angegebene Bedeutung haben,

mit Iso(thio)cyanaten der allgemeinen Formel (III)

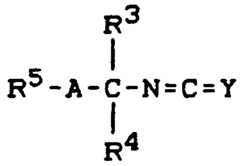

(III)

in welcher

A, $R^3$, $R^4$, $R^5$ und Y die oben angegebene Bedeutung haben,

gegebenenfalls in Gegenwart eines Verdünnungsmittels und gegebenenfalls in Gegenwert eines Reaktionshilfsmittels umsetzt, oder wenn man

(b) Triazolinone der allgemeinen Formel (IV)

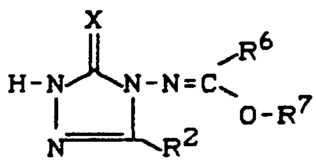

(IV)

in welcher

$R^2$ und X die oben angegebene Bedeutung haben und

$R^6$ und $R^7$ für Alkyl, Alkenyl, Alkinyl oder Cycloalkyl stehen, sowie $R^6$ auch für Wasserstoff steht,

mit Iso(thio)cyanaten der allgemeinen Formel (III)

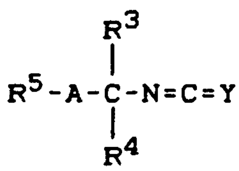

(III)

in welcher

A, $R^3$, $R^4$, $R^5$ und Y die oben angegebene Bedeutung haben,

gegebenenfalls in Gegenwart eines Verdünnungsmittels und gegebenenfalls in Gegenwart eines Reaktionshilfsmittels umsetzt und die so erhaltenen Verbindungen der allgemeinen Formel (V)

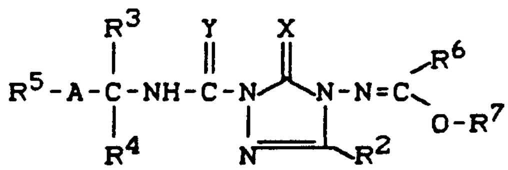

(V)

in welcher

A, $R^2$, $R^3$, $R^4$, $R^5$, $R^6$, $R^7$, X und Y die oben angegebene Bedeutung haben,

in einem zweiten Reaktionsschritt mit einem Reduktionsmittel der Formel (VI) oder (VII)

$MBH_4$ (VI)

$MAlH_4$ (VII)

worin

M für ein Alkalimetallatom steht,

gegebenenfalls in Gegenwart eines Verdünnungsmittels zu den Verbindungen der allgemeinen Formel

(VIII)

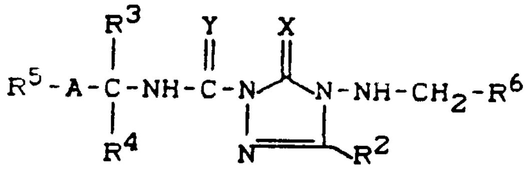

(VIII)

in welcher

A, $R^2$, $R^3$, $R^4$, $R^5$, $R^6$, X und Y die oben angegebene Bedeutung haben,

umsetzt, oder wenn man

(c) Aminotriazolinone der allgemeinen Formel (IX)

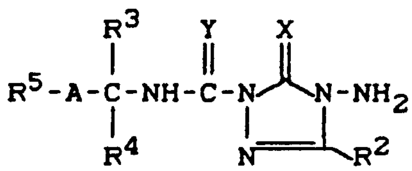

(IX)

in welcher

A, $R^2$, $R^3$, $R^4$, $R^5$, X und Y die oben angegebene Bedeutung haben,

mit Orthocarbonsäureestern der allgemeinen Formel (X)

$R^6$-C($OR^7$)$_3$ (X)

in welcher

$R^6$ und $R^7$ die oben angegebene Bedeutung haben,

gegebenenfalls in Gegenwart eines Verdünnungsmittels und gegebenenfalls in Gegenwart eines Reaktionshilfsmittels zu Verbindungen der allgemeinen Formel (V)

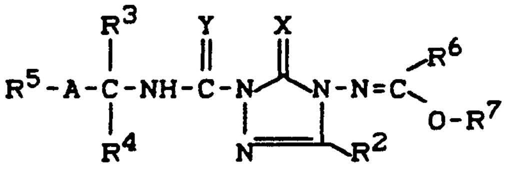

(V)

in welcher

A, $R^2$, $R^3$, $R^4$, $R^5$, $R^6$, $R^7$, X und Y die oben angegebene Bedeutung haben,

umsetzt und diese Verbindungen der Formel (V) in einem zweiten Reaktionsschritt mit einem Reduktionsmittel der Formel (VI) oder (VII)

$MBH_4$ (VI)

$MAlH_4$ (VII)

worin

M für ein Alkalimetallatom steht,

gegebenenfalls in Gegenwart eines Verdünnungsmittels zu den Verbindungen der allgemeinen Formel (VIII)

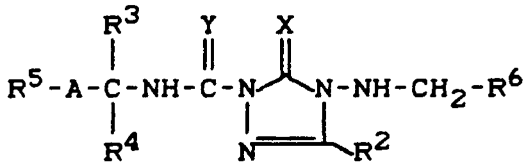 (VIII)

in welcher
A, $R^2$, $R^3$, $R^4$, $R^5$, $R^6$, X und Y die oben angegebene Bedeutung haben,
umsetzt, oder wenn man
(d) Triazolinone der allgemeinen Formel (II)

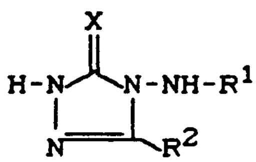 (II)

in welcher
$R^1$, $R^2$ und X die oben angegebene Bedeutung haben,
mit Chlor(thio)kohlensäurederivaten der allgemeinen Formel (XI)

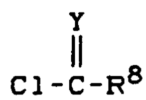 (XI)

in welcher
Y die oben angegebene Bedeutung hat und
$R^8$ für Alkoxy, Aryloxy, Aralkyloxy oder Halogen steht,
gegebenenfalls in Gegenwart eines Verdünnungsmittels und gegebenenfalls in Gegenwart eines Reaktionshilfsmittels umsetzt und die so erhältlichen Verbindungen der allgemeinen Formel (XII)

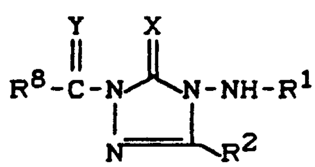 (XII)

in welcher
$R^1$, $R^2$, $R^8$, X und Y die oben angegebene Bedeutung haben,
in einer anschließenden zweiten Stufe mit Aminen der allgemeinen Formel (XIII)

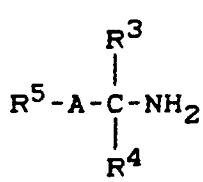 (XIII)

in welcher

A, $R^3$, $R^4$ und $R^5$ die oben angegebene Bedeutung haben,

gegebenenfalls in Gegenwart eines Verdünnungsmittels und gegebenenfalls in Gegenwart eines Reaktionshilfsmittels zu Verbindungen der Formel (I) umsetzt, oder wenn man

(e) Triazolinone der allgemeinen Formel (II)

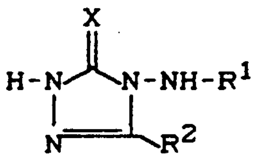

(II)

in welcher

$R^1$, $R^2$ und X die oben angegebene Bedeutung haben,

mit (Thio)Carbamidsäurederivaten der allgemeinen Formel (XIV)

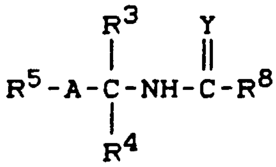

(XIV)

in welcher

A, $R^3$, $R^4$, $R^5$, $R^8$ und Y die oben angegebene Bedeutung haben,

gegebenenfalls in Gegenwart eines Verdünnungsmittels und gegebenenfalls in Gegenwart eines Reaktionshilfsmittels umsetzt, oder wenn man

(f) Triazolinon-Hydrazone der allgemeinen Formel (XV)

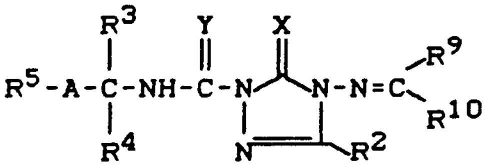

(XV)

in welcher

A, $R^2$, $R^3$, $R^4$, $R^5$, X und Y die oben angegebene Bedeutung haben und

$R^9$ und $R^{10}$ für Wasserstoff, Alkyl, Alkenyl, Alkinyl oder Cycloalkyl stehen,

mit Reduktionsmitteln, gegebenenfalls in Gegenwart von Katalysatoren und gegebenenfalls in Gegenwart von Verdünnungsmitteln umsetzt.

Schließlich wurde gefunden, daß die neuen substituierten Triazolinone der allgemeinen Formel (I) interessante herbizide Eigenschaften besitzen.

Überraschenderweise zeigen die erfindungsgemäßen substituierten Triazolinone der allgemeinen Formel (I) wesentlich bessere herbizide Wirkung als aus dem Stand der Technik bekannte ähnliche Triazolinone, wie z.B. 4-Amino-1-(N-phenyl-carbamoyl)-3-methyl-triazolin-5-on.

In den Definitionen stehen die aliphatischen Kohlenstoffketten, wie Alkyl, Alkenyl, Alkinyl, Alkandiyl, Alkendiyl und Alkindiyl, für jeweils geradkettige oder verzweigte Gruppierungen, auch wenn dies nicht in jedem Fall ausdrücklich angegeben ist. Aryl steht vorzugsweise für Naphthyl oder Phenyl, insbesondere für Phenyl. Die erfindungsgemäßen substituierten Triazolinone sind durch die Formel (I) allgemein definiert. Bevorzugt sind diejenigen Verbindungen der Formel (I), bei welchen

A für jeweils geradkettiges oder verzweigtes Alkandiyl mit 1 bis 6 Kohlenstoffatomen, Alkendiyl mit 2 bis 6 Kohlenstoffatomen oder Alkindiyl mit 2 bis 6 Kohlenstoffatomen steht,

$R^1$ für jeweils geradkettiges oder verzweigtes Alkyl mit 1 bis 8 Kohlenstoffatomen, Alkenyl mit 2

bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Cyanalkyl oder Alkoxyalkyl mit 1 bis 6 Kohlenstoffatomen in den Alkyteilen, Cycloalkyl mit 3 bis 6 Kohlenstoffatomen, Halogencycloalkyl mit 3 bis 6 Kohlenstoffatomen und 1 bis 4 Halogenatomen, Alkylcycloalkyl oder Cycloalkylalkyl mit jeweils 1 bis 4 Kohlenstoffatomen im Alkylteil und 3 bis 6 Kohlenstoffatomen im Cycloalkylteil steht,

$R^2$ für jeweils geradkettiges oder verzweigtes Alkyl mit 1 bis 8 Kohlenstoffatomen, Halogenalkyl mit 1 bis 8 Kohlenstoffatomen und 1 bis 17 gleichen oder verschiedenen Halogenatomen, Alkoxyalkyl mit 1 bis 6 Kohlenstoffatomen in den Alkylteilen oder Cycloalkyl mit 3 bis 6 Kohlenstoffatomen steht,

$R^3$ für Wasserstoff oder für geradkettiges oder verzweigtes Alkyl mit 1 bis 4 Kohlenstoffatomen steht,

$R^4$ für geradkettiges oder verzweigtes Alkyl mit 1 bis 4 Kohlenstoffatomen steht,

$R^5$ für jeweils gegebenenfalls substituiertes Phenyl, Naphthyl oder Cyclohexyl steht, wobei für Phenyl als mögliche Substituenten vorzugsweise Halogen, Cyano, Nitro, Amino, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Phenyl, Phenoxy, Cyclohexyl oder gegebenenfalls durch Fluor und/oder Chlor bis zu vierfach substituiertes Alkylendioxy mit 1 oder 2 Kohlenstoffatomen ausgewählt sind und für Naphthyl oder Cyclohexyl als mögliche Substituenten geradkettige oder verzweigte Alkylgruppen mit 1 bis 4 Kohlenstoffatomen infrage kommen, und

X und Y für Sauerstoff oder Schwefel stehen, wobei folgende Verbindungen ausgenommen sind: 2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

Besonders bevorzugt sind die Verbindungen der Formel (I), in welcher

A für Methan-1,1-diyl (Methylen), Ethan-1,2-diyl (Ethylen, Dimethylen), Propan-1,2-diyl, Propan-1,3-diyl, Ethan-1,1-diyl, Propan-2,2-diyl, Ethen-1,2-diyl (Ethenylen, Vinylen) oder Ethin-1,2-diyl (Ethinylen) steht,

$R^1$ für Methyl, Ethyl, Propyl, Isopropyl, Butyl, Isobutyl, sec-Butyl oder tert-Butyl steht,

$R^2$ für Methyl, Ethyl, Propyl, Isopropyl, Butyl, Isobutyl, sec-Butyl, tert-Butyl, Fluormethyl, Chlormethyl, Difluormethyl, Dichlormethyl, Chlorfluormethyl, Trifluormethyl, Trichlormethyl, Chlordifluormethyl, Dichlorfluormethyl, Methoxymethyl, Methoxyethyl, Ethoxymethyl oder Cyclopropyl steht,

$R^3$ für Wasserstoff, Methyl, Ethyl, Propyl, Isopropyl, Butyl oder Isobutyl steht,

$R^4$ für Methyl, Ethyl, Propyl, Isopropyl, Butyl oder Isobutyl steht,

$R^5$ für gegebenenfalls durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Difluormethyl, Trifluormethyl, Difluormethoxy, Trifluormethoxy, Difluormethylthio, Trifluormethylthio, Trifluormethylsulfinyl, Trifluormethylsulfonyl, Methylsulfinyl, Methylsulfonyl, Cyclohexyl, Phenyl oder Phenoxy substituiertes Phenyl oder für gegebenenfalls durch Methyl, Ethyl, Propyl, Isopropyl, Butyl, Isobutyl, sec-Butyl oder tert-Butyl substituiertes Cyclohexyl steht,

X für Sauerstoff oder Schwefel steht und

Y für Sauerstoff oder Schwefel steht, wobei folgende Verbindungen ausgenommen sind:

2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

Ganz besonders bevorzugt sind die Verbindungen der Formel (I), in welcher

A für Methan-1,1-diyl, Ethan-1,2-diyl, Propan-1,3-diyl oder Ethin-1,2-diyl steht,

$R^1$ für Methyl oder Ethyl steht,

$R^2$ für Methyl, Ethyl, Propyl, Isopropyl oder Cyclopropyl steht,

$R^3$ für Wasserstoff, Methyl oder Ethyl steht,

$R^4$ für Methyl oder Ethyl steht,

$R^5$ für gegebenenfalls durch Fluor, Chlor, Brom, Methyl, Ethyl, Methoxy, Ethoxy, Trifluormethyl, Difluormethoxy, Trifluormethoxy, Methylthio, Methylsulfinyl, Methylsulfonyl, Trifluormethylthio, Trifluormethylsulfinyl oder Trifluormethylsulfonyl substituiertes Phenyl oder für Cyclohexyl steht,

X für Sauerstoff steht und

Y für Sauerstoff oder Schwefel steht, wobei folgende Verbindungen ausgenommen sind: 2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

Die oben aufgeführten allgemeinen oder in Vorzugsbereichen aufgeführten Restedefinitionen können untereinander, also auch zwischen den jeweiligen Vorzugsbereichen beliebig kombiniert werden.

Beispiele für die Verbindungen der Formel (I) sind in der nachstehenden Tabelle 1 aufgeführt:

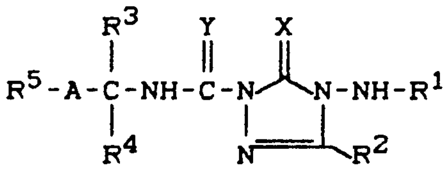

(I)

Tabelle 1: Beispiele für die Verbindungen der Formel (I)

| A | $R^1$ | $R^2$ | $R^3$ | $R^4$ | $R^5$ | X | Y |
|---|---|---|---|---|---|---|---|
| $-CH_2-$ | $CH_3$ | $CH_3$ | H | $CH_3$ | 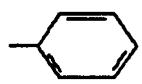 | O | O |
| $-CH_2CH_2-$ | $CH_3$ | $CH_3$ | H | $CH_3$ | 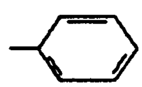 | O | O |
| $-C{\equiv}C-$ | $CH_3$ | $CH_3$ | H | $CH_3$ | 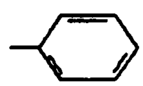 | O | O |

<u>Tabelle 1</u> - Fortsetzung

| A | $R^1$ | $R^2$ | $R^3$ | $R^4$ | $R^5$ | X | Y |
|---|---|---|---|---|---|---|---|
| $-CH_2-$ | $CH_3$ | $CH_3$ | H | $CH_3$ | 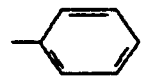 | O | S |
| $-CH_2-$ | $CH_3$ | $CH_3$ | $CH_3$ | $CH_3$ | 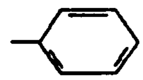 | O | O |
| $-CH_2-$ | $C_2H_5$ | $CH_3$ | H | $CH_3$ | 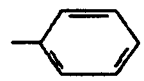 | O | O |
| $-CH_2-$ | $CH_3$ | $C_2H_5$ | H | $CH_3$ | 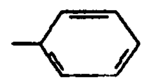 | O | O |
| $-CH_2CH_2-$ | $CH_3$ | $C_2H_5$ | H | $CH_3$ | 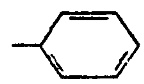 | O | O |
| $-CH_2CH_2-$ | $CH_3$ | 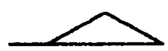 | H | $CH_3$ | 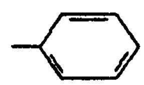 | O | O |
| $-CH_2CH_2-$ | $CH_3$ | 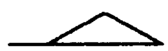 | $CH_3$ | $CH_3$ | 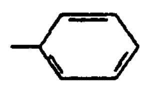 | O | O |
| $-CH_2-$ | $CH_3$ | 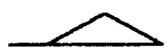 | $CH_3$ | $C_2H_5$ | 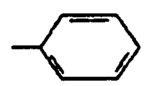 | O | O |
| $-CH_2CH_2-$ | $C_2H_5$ | $C_2H_5$ | H | $CH_3$ | 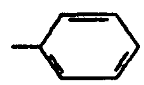 | O | O |
| $-CH_2CH_2-$ | $C_2H_5$ | $C_2H_5$ | $CH_3$ | $CH_3$ | 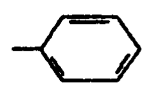 | O | O |
| $-CH_2CH_2-$ | $CH_3$ | $CH_3$ | $C_2H_5$ | $C_2H_5$ | 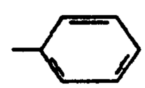 | O | O |

Tabelle 1 - Fortsetzung

| A | $R^1$ | $R^2$ | $R^3$ | $R^4$ | $R^5$ | X | Y |
|---|---|---|---|---|---|---|---|
| $-CH_2-$ | $CH_3$ | $CH_3$ | H | $CH_3$ | 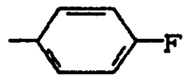 | O | O |
| $-CH_2CH_2-$ | $CH_3$ | $CH_3$ | H | $CH_3$ | 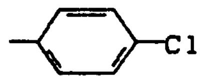 | O | O |
| $-CH_2CH_2-$ | $CH_3$ | $CH_3$ | $CH_3$ | $CH_3$ | 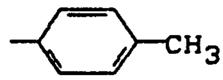 | O | O |
| $-C{\equiv}C-$ | $CH_3$ | $C_2H_5$ | $CH_3$ | $CH_3$ | 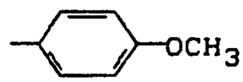 | O | O |
| $-CH_2-$ | $CH_3$ | 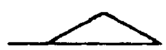 | H | $CH_3$ | 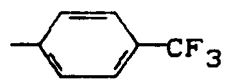 | O | O |
| $-CH_2CH_2-$ | $C_2H_5$ | $CH_3$ | $CH_3$ | $CH_3$ | 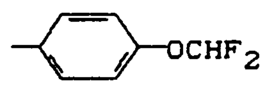 | O | O |
| $-CH_2CH_2-$ | $CH_3$ | $CH_3$ | $CH_3$ | $CH_3$ | 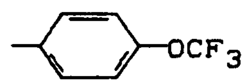 | O | O |
| $-CH_2CH_2-$ | $CH_3$ | $C_2H_5$ | H | $C_2H_5$ | 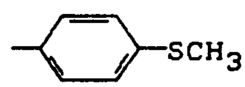 | O | O |
| $-CH_2-$ | $CH_3$ | $CH_3$ | $CH_3$ | $CH_3$ | 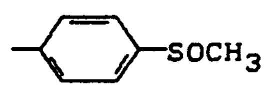 | O | O |
| $-CH_2CH_2-$ | $CH_3$ | $CH_3$ | $CH_3$ | $CH_3$ | 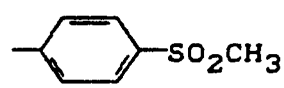 | O | O |

Tabelle 1 - Fortsetzung

| A | $R^1$ | $R^2$ | $R^3$ | $R^4$ | $R^5$ | X | Y |
|---|---|---|---|---|---|---|---|
| $-CH_2-$ | $C_2H_5$ | $C_2H_5$ | H | $CH_3$ | 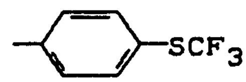 | O | O |
| $-CH_2CH_2-$ | $CH_3$ | $CH_3$ | H | $CH_3$ | 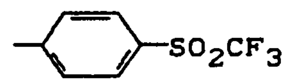 | O | O |

Verwendet man beispielsweise 1-Methyl-3-phenyl-propyl-isocyanat und 4-Methylamino-5-methyl-2,4-dihydro-3H-1,2,4-triazol-3-on als Ausgangsstoffe, so läßt sich der Reaktionsablauf des erfindungsgemäßen Verfahrens (a) durch das folgende Formelschema darstellen:

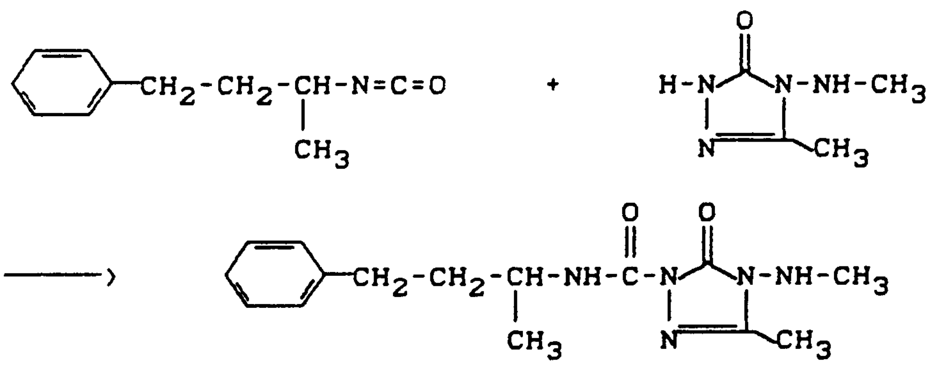

Verwendet man beispielsweise 1,1-Dimethyl-3-phenyl-propyl-isocyanat und 4-Ethoxymethylenimino-5-isopropyl-2,4-dihydro-3H-1,2,4-triazol-3-on als Ausgangsstoffe, so läßt sich der Reaktionsablauf des erfindungsgemäßen Verfahrens (b) durch das folgende Formelschema darstellen:

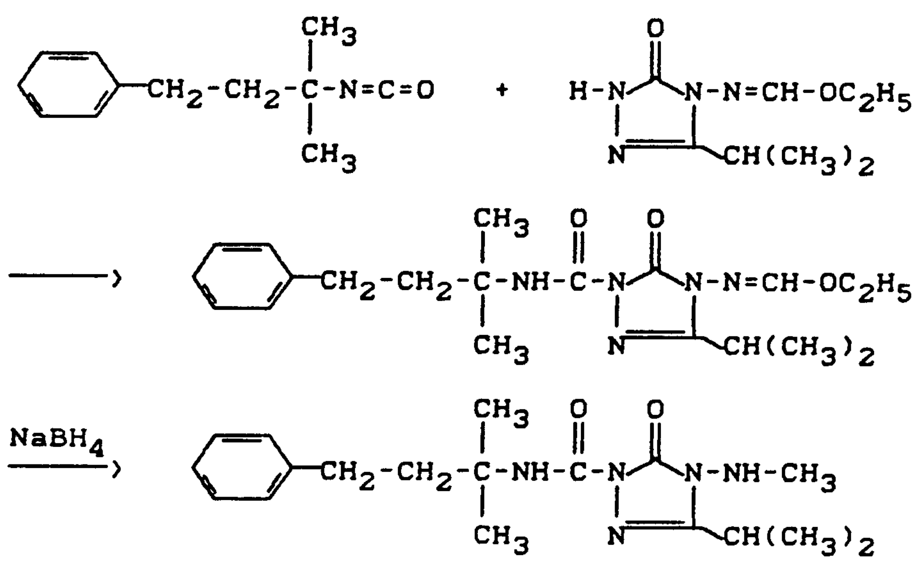


Verwendet man beispielsweise 2-(1-Methyl-2-phenyl-ethylaminocarbonyl)-4-amino-5-ethyl-2,4-dihydro-3H-1,2,4-triazol-3-on und Orthoessigsäure-triethylester als Ausgangsstoffe, so läßt sich der Reaktionsablauf des erfindungsgemäßen Verfahrens (c) durch das folgende Formelschema darstellen:

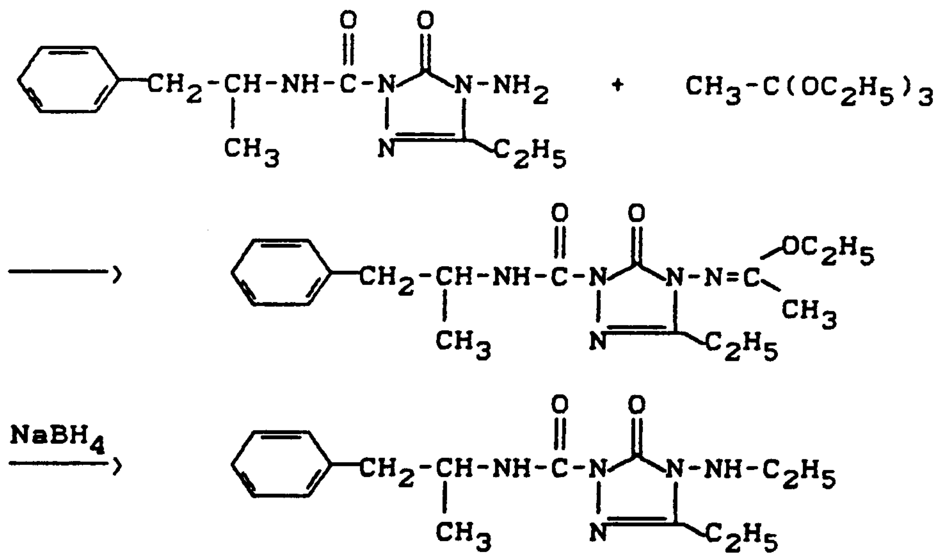


Verwendet man beispielsweise 4-Methylamino-5-propyl-2,4-dihydro-3H-1,2,4-triazol-3-on, Chlorthioameisensäure-O-phenylester und 1,1-Dimethyl-3-cyclohexyl-propylamin als Ausgangsstoffe, so läßt sich der Reaktionsverlauf beim erfindungsgemäßen Verfahren (d) durch das folgende Formelschema darstellen:

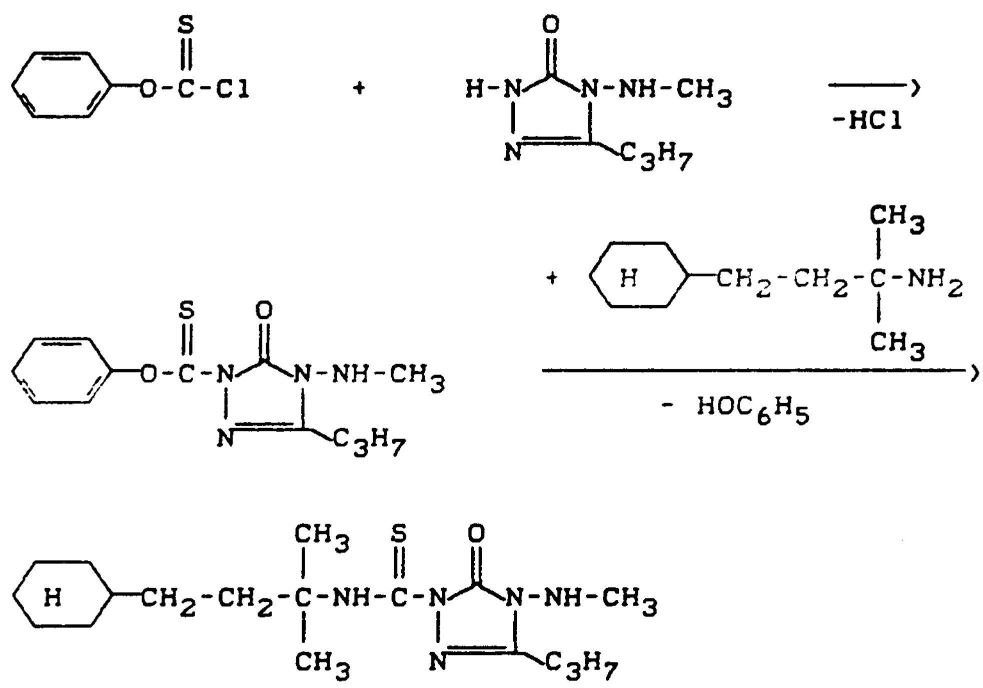


Verwendet man beispielsweise 4-Ethylamino-5-methyl-2,4-dihydro-3H-1,2,4-triazol-3-on und N-(1,1-Dimethyl-2-phenyl-ethyl)-O-phenyl-urethan als Ausgangsstoffe, so läßt sich der Reaktionsverlauf des erfindungsgemäßen Verfahrens (e) durch das folgende Formelschema darstellen:

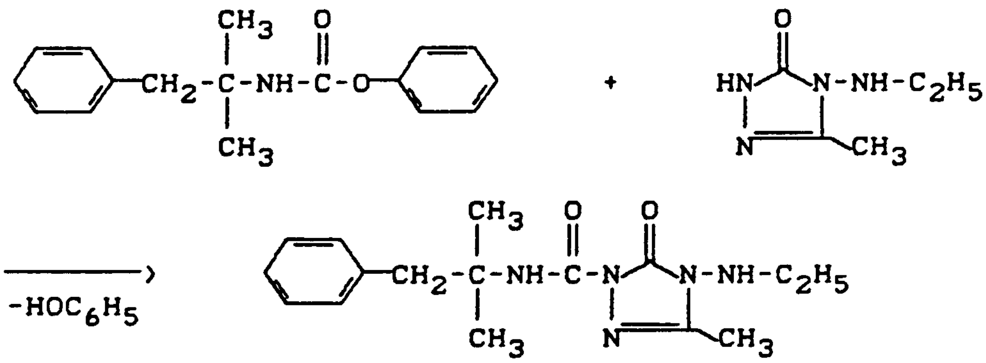


Verwendet man beispielsweise 2-(1-Ethyl-3-phenyl-propylaminocarbonyl)-4-isopropylidenimino-5-methyl-2,4-dihydro-3H-1,2,4-triazol-3-on und Natriumcyanoborhydrid als Ausgangsstoffe, so läßt sich der Reaktionsverlauf des erfindungsgemäßen Verfahrens (f) durch das folgende Formelschema darstellen:

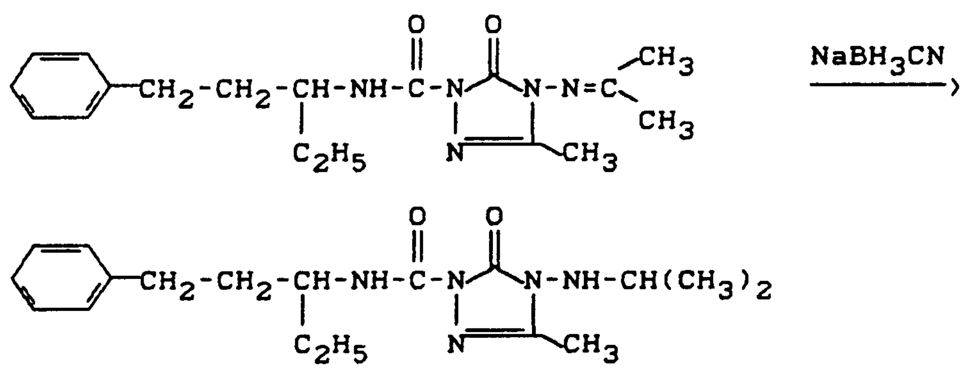


Die bei den erfindungsgemäßen Verfahren (a), (d) und (e) zur Herstellung von Verbindungen der Formel (I) als Ausgangsstoffe zu verwendenden Triazolinone sind durch die Formel (II) allgemein definiert.

In Formel (II) haben $R^1$, $R^2$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und X angegeben wurden.

Die Ausgangsstoffe der Formel (II) sind bekannt und/oder können nach an sich bekannten Verfahren hergestellt werden (vgl. EP-A 399 294).

Die bei den erfindungsgemäßen Verfahren (a) und (b) zur Herstellung von Verbindungen der Formel (I) als Ausgangsstoffe zu verwendenden Iso(thio)cyanate sind durch die Formel (III) allgemein definiert.

In Formel (III) haben A, $R^3$, $R^4$, $R^5$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^3$, $R^4$, $R^5$ und Y angegeben wurden.

Die Ausgangsstoffe der Formel (III) sind weitgehend bekannte organische Synthesechemikalien (vgl. Nouv. J. Chim. 1 (1977), 243-254 - zitiert in Chem. Abstracts 87: 151614a; Liebigs Ann. Chem. 562 (1949), 75-136; DE-P 41 03 700.6 vom 07. 02. 91/LeA 28217; Herstellungsbeispiele).

Man erhält die Verbindungen der Formel (III), wenn man Aminoverbindungen der Formel (XIII)

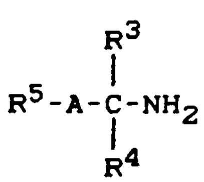

(XIII)

in welcher

A, $R^3$, $R^4$ und $R^5$ die oben angegebene Bedeutung haben,

mit Phosgen, gegebenenfalls in Gegenwart eines Verdünnungsmittels, wie z.B. Toluol oder Chlorbenzol, bei Temperaturen zwischen 0 °C und 150 °C umsetzt, oder wenn man die als Ausgangsstoffe angegebenen Aminoverbindungen der Formel (XIII) mit Thiophosgen, gegebenenfalls in Gegenwart von Verdünnungsmitteln, wie z.B. Toluol oder Chloroform und Wasser, bei Temperaturen zwischen -10 °C und +50 °C umsetzt.

Die beim erfindungsgemäßen Verfahren (b) zur Herstellung von Verbindungen der Formel (I) als Ausgangsstoffe zu verwendenden Triazolinone sind durch die Formel (IV) allgemein definiert.

In Formel (IV) haben $R^2$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ und X angegeben wurden;

$R^6$ steht vorzugsweise für Wasserstoff, $C_1$-$C_4$-Alkyl oder Cyclopropyl und

$R^7$ steht vorzugsweise für $C_1$-$C_4$-Alkyl.

Die Ausgangsstoffe der Formel (IV) sind bekannt und/oder können nach an sich bekannten Verfahren hergestellt werden (vgl. J. Heterocycl. Chem. 17 (1980), 1691-1696; EP-A 399294).

Die in der zweiten Stufe der erfindungsgemäßen Verfahren (b) und (c) zu verwendenden Reduktionsmittel sind durch die Formeln (VI) und (VII) allgemein definiert. In den Formeln (VI) und (VII) steht M jeweils vorzugsweise für Lithium, Natrium oder Kalium, insbesondere für Lithium oder Natrium.

Die Reduktionsmittel der Formeln (VI) und (VII) sind bekannte Synthesechemikalien.

Die nach Verfahren (b) oder (c) herzustellenden Verbindungen der Formel (VIII) stellen eine Auswahl aus den erfindungsgemäßen Verbindungen der Formel (I) dar und sind somit ebenfalls erfindungsgemäße neue Stoffe.

Die beim erfindungsgemäßen Verfahren (c) zur Herstellung von Verbindungen der Formel (I) als Ausgangsstoffe zu verwendenden Aminotriazolinone sind durch die Formel (IX) allgemein definiert.

In Formel (IX) haben A, $R^2$, $R^3$, $R^4$, $R^5$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^2$, $R^3$, $R^4$, $R^5$, X und Y angegeben wurden.

Die Ausgangsstoffe der Formel (IX) sind bekannt und/oder können nach an sich bekannten Verfahren hergestellt werden (vgl. EP-A 299466).

Die beim erfindungsgemäßen Verfahren (c) zur Herstellung von Verbindungen der Formel (I) weiter als Ausgangsstoffe zu verwendenden Orthocarbonsäureester sind durch die Formel (X) allgemein definiert.

In Formel (X) haben $R^6$ und $R^7$ vorzugsweise diejenige Bedeutung, die bereits oben im Zusammenhang mit der Beschreibung der Verbindungen der Formel (IV) vorzugsweise für $R^6$ und $R^7$ angegeben wurde.

Die Orthocarbonsäureester der Formel (X) sind bekannte organische Synthesechemikalien.

Die beim erfindungsgemäßen Verfahren (d) zur Herstellung von Verbindungen der Formel (I) als Ausgangsstoffe zu verwendenden Chlor(thio)kohlensäurederivate sind durch die Formel (XI) allgemein definiert.

In Formel (XI) steht $R^8$ vorzugsweise für $C_1$-$C_4$-Alkoxy, Phenyloxy, Benzyloxy oder Chlor.

Die Ausgangsstoffe der Formel (XI) sind bekannte organische Synthesechemikalien.

Die beim erfindungsgemäßen Verfahren (d) in der zweiten Stufe als Ausgangsstoffe zu verwendenden Amine sind durch die Formel (XIII) allgemein definiert.

In Formel (XIII) haben A, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^3$, $R^4$ und $R^5$ angegeben wurden.

Die Ausgangsstoffe der Formel (XIII) sind bekannt und/oder können nach an sich bekannten Verfahren hergestellt werden (vgl. J Med. Chem. 25 (1982), 1363-1370; Tetrahedron Lett. 29 (1988), 223-224; Chem. Ber. 117 (1984), 856-858; DE-OS 3426919; EP-A 237305; J. Am. Chem. Soc. 71 (1949), 3482-3485; Tetrahedron Lett. 27 (1986), 3957-3960; Bull. Soc. Chim. France 1974 (3-4, Pt. 2), 615-622; Tetrahedron Lett. 31 (1990), 2661-2664; J. Med. Chem. 13 (1970), 1249-1250).

Die beim erfindungsgemäßen Verfahren (e) zur Herstellung von Verbindungen der Formel (I) als Ausgangsstoffe zu verwendenden (Thio)Carbamidsäurederivate sind durch die Formel (XIV) allgemein definiert.

In Formel (XIV) haben A, $R^3$, $R^4$, $R^5$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^3$, $R^4$, $R^5$ und Y angegeben wurden;

$R^8$ steht vorzugsweise für $C_1$-$C_4$-Alkoxy, Phenyloxy, Benzyloxy oder Chlor.

Die Ausgangsstoffe der Formel (XIV) sind bekannte organische Chemikalien.

Die beim erfindungsgemäßen Verfahren (f) zur Herstellung von Verbindungen der Formel (I) als

Ausgangsstoffe zu verwendenden Triazolinon-Hydrazone sind durch die Formel (XV) allgemein definiert.

In Formel (XV) haben A, $R^2$, $R^3$, $R^4$, $R^5$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^2$, $R^3$, $R^4$, $R^5$, X und Y angegeben wurden;

$R^9$ und $R^{10}$ stehen vorzugsweise jeweils unabhängig voneinander für Wasserstoff oder $C_1$-$C_4$-Alkyl.

Die Ausgangsstoffe der Formel (XV) sind bekannt und/oder können nach an sich bekannten Verfahren hergestellt werden (vgl. EP-A 294666).

Das erfindungsgemäße Verfahren (a) wird vorzugsweise in Gegenwart eines Verdünnungsmittels durchgeführt.

Als Verdünnungsmittel zur Durchführung des erfindungsgemäßen Verfahrens (a) kommen insbesondere inerte organische Lösungsmittel infrage. Hierzu gehören beispielsweise aliphatische, alicyclische oder aromatische, gegebenenfalls halogenierte Kohlenwasserstoffe, wie Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (a) wird gegebenenfalls in Gegenwart eines geeigneten Reaktionshilfsmittels durchgeführt. Als solche kommen alle üblichen anorganischen oder organischen Basen in Frage. Hierzu gehören beispielsweise tertiäre Amine, wie Triethylamin, N,N-Dimethylanilin, N,N-Diethylbenzylamin, N,N-Dimethylcyclohexylamin oder Dibutylzinndilaureat, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) setzt man pro Mol an Triazolinon der Formel (II) im allgemeinen 1.0 bis 2.0 Mol, vorzugsweise 1.0 bis 1.5 Mol an Iso(thio)cyanat der Formel (III) und gegebenenfalls 0.001 bis 2.0 Mol, vorzugsweise 0.001 bis 1.0 Mol an Reaktionshilfsmittel ein. Die Reaktionsdurchführung, Aufarbeitung und Isolierung der Reaktionsprodukte erfolgt nach allgemein üblichen Methoden.

Die erste Stufe des erfindungsgemäßen Verfahrens (b) wird vorzugsweise in Gegenwart eines Verdünnungsmittels durchgeführt. Es können hierbei die gleichen Lösungsmittel verwendet werden, die oben für das erfindungsgemäße Verfahren (a) angegeben sind.

Auch bezüglich der Verwendung eines Reaktionshilfsmittels und der Reaktionstemperaturen gelten bei Verfahren (b) in der ersten Stufe die oben für Verfahren (a) gemachten Angaben.

Zur Durchführung des erfindungsgemäßen Verfahrens (b) setzt man auf 1 Mol Triazolinon der Formel (IV) im allgemeinen 1,0 bis 2,0 Mol, vorzugsweise 1,0 bis 1,5 Mol, Iso(thio)cyanat der Formel (III) und gegebenenfalls 0,001 bis 2,0 Mol, vorzugsweise 0,01 bis 1,0 Mol, Reaktionshilfsmittel ein.

Die Reaktionsdurchführung und gegebenenfalls die Aufarbeitung und Isolierung der Intermediate der Formel (V) kann nach allgemein üblichen Methoden erfolgen.

Die zweite Stufe des erfindungsgemäßen Verfahrens (b) wird vorzugsweise in Gegenwart eines polaren Lösungsmittels durchgeführt. Als solche kommen vorzugsweise Wasser, Alkohole wie Methanol, Ethanol, Propanol, Isopropanol, Butanol, Isobutanol, sec-Butanol und tert-Butanol, Etheralkohole wie Methoxyethanol und Ethoxyethanol, oder Ether wie Diethylether, Dipropylether, Diisopropylether, Tetrahydrofuran und Dioxan, in Betracht.

Die Reaktionstemperaturen können bei der zweiten Stufe von Verfahren (b) in einem größeren Bereich variiert werden. Im allgemeinen arbeitet man bei Temperaturen zwischen -20 °C und +100 °C, vorzugsweise zwischen 0 °C und +30 °C.

Zur Durchführung der zweiten Stufe von Verfahren (b) setzt man auf 1 Mol Intermediat der Formel (V) im allgemeinen 0,5 bis 5 Mol, vorzugsweise 1 bis 3 Mol, Reduktionsmittel der Formel (VI) oder (VII) ein.

Die Reaktionsdurchführung, Aufarbeitung und Isolierung der Reaktionsprodukte erfolgt nach allgemein üblichen Methoden.

Die erste Stufe des erfindungsgemäßen Verfahrens (c) wird gegebenenfalls in Gegenwart eines Verdünnungsmittels durchgeführt. Es können die gleichen Lösungsmittel verwendet werden, die oben für das erfindungsgemäße Verfahren (a) angegeben sind.

Verfahren (c) wird in der ersten Stufe vorzugsweise in Gegenwart eines Reaktionshilfsmittels durchgeführt. Als solche sind hierbei insbesondere starke Säuren, wie z.B. Hydrogenchlorid, Schwefelsäure, Methansulfonsäure, Benzolsulfonsäure und p-Toluolsulfonsäure, geeignet.

Die Reaktionstemperaturen können in der ersten Stufe des erfindungsgemäßen Verfahrens (c) in einem größeren Bereich variiert werden. Im allgemeinen arbeitet man bei Temperaturen zwischen 0 °C und 200 °C, vorzugsweise bei Temperaturen zwischen 50 °C und 150 °C.

Zur Durchführung des erfindungsgemäßen Verfahrens (c) setzt man auf 1 Mol Amino-triazolinon der Formel (IX) im allgemeinen 1 bis 100 Mol, vorzugsweise 1 bis 20 Mol, Orthocarbonsäureester der Formel (X) und gegebenenfalls 0,001 bis 2,0 Mol, vorzugsweise 0,01 bis 1,0 Mol, Reaktionshilfsmittel ein.
Die Reaktionsdurchführung und gegebenenfalls die Aufarbeitung und Isolierung der Intermediate der Formel (V) kann nach allgemein üblichen Methoden erfolgen.

Die zweite Stufe des erfindungsgemäßen Verfahrens (c) wird vorzugsweise in Gegenwart eines Verdünnungsmittels durchgeführt. Es können hierbei die gleichen Lösungsmittel verwendet werden, die oben für die zweite Stufe von Verfahren (b) angegeben sind.

Die Reaktionstemperaturen können bei der zweiten Stufe von Verfahren (c) in einem größeren Bereich variiert werden. Im allgemeinen arbeitet man bei Temperaturen zwischen -20 °C und +100 °C, vorzugsweise zwischen 0 °C und +30 °C.

Zur Durchführung von Verfahren (c) setzt man in der zweiten Stufe auf 1 Mol Intermediat der Formel (V) im allgemeinen 0,5 bis 5 Mol, vorzugsweise 1 bis 3 Mol, Reduktionsmittel der Formel (VI) oder (VII) ein.

Die Reaktionsdurchführung, Aufarbeitung und Isolierung der Reaktionsprodukte erfolgt nach üblichen Methoden.

Das erfindungsgemäße Verfahren (d) wird in beiden Stufen vorzugsweise in Gegenwart eines Verdünnungsmittels durchgeführt. Es können hierbei die gleichen Lösungsmittel verwendet werden, die oben für das erfindungsgemäße Verfahren (a) angegeben sind.

Das erfindungsgemäße Verfahren (d) kann in beiden Stufen gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der beiden Stufen des erfindungsgemäßen Verfahrens (d) in einem größeren Bereich variiert werden. Im allgemeinen arbeitet man bei Temperaturen zwischen 0 °C und 150 °C, vorzugsweise zwischen 10 °C und 100 °C.

Zur Durchführung des erfindungsgemäßen Verfahrens (d) setzt man auf 1 Mol Triazolinon der Formel (II) im allgemeinen 1 bis 2 Mol, vorzugsweise 1,0 bis 1,5 Mol, (Thio)Chlorkohlensäurederivat der Formel (XI) und 1 bis 2 Mol, vorzugsweise 1,0 bis 1,5 Mol, Reaktionshilfsmittel ein.

Die Reaktionsdurchführung und gegebenenfalls die Aufarbeitung und Isolierung der Intermediate der Formel (XII) kann nach allgemein üblichen Methoden erfolgen.

Zur Durchführung der zweiten Stufe von Verfahren (d) setzt man auf 1 Mol Intermediat der Formel (XII) im allgemeinen 1 bis 2 Mol, vorzugsweise 1,0 bis 1,5 Mol, Amin der Formel (XIII) ein.

Die Reaktionsdurchführung, Aufarbeitung und Isolierung der Produkte der Formel (I) erfolgt nach allgemein üblichen Methoden.

Das erfindungsgemäße Verfahren (e) wird vorzugsweise in Gegenwart eines Verdünnungsmittels durchgeführt. Es können hierbei die gleichen Lösungsmittel verwendet werden, die oben für das erfindungsgemäße Verfahren (a) angegeben sind.

Verfahren (e) wird vorzugsweise in Gegenwart eines Reaktionshilfsmittels durchgeführt. Es können hierbei die gleichen Reaktionshilfsmittel eingesetzt werden, die oben für das erfindungsgemäße Verfahren (d) angegeben sind.

Die Reaktionstemperaturen können bei dem erfindungsgemäßen Verfahren (e) in einem größeren Bereich variiert werden. Im allgemeinen arbeitet man bei Temperaturen zwischen 0 °C und 150 °C, vorzugsweise bei Temperaturen zwischen 10 °C und 120 °C.

Zur Durchführung des erfindungsgemäßen Verfahrens (e) setzt man auf 1 Mol Triazolinon der Formel (II) im allgemeinen 1 bis 2 Mol, vorzugsweise 1,0 bis 1,5 Mol, (Thio)Carbamidsäurederivat der Formel (XIV) ein.

Die Reaktionsdurchführung, Aufarbeitung und Isolierung der Produkte der Formel (I) erfolgt nach allgemein üblichen Methoden.

Das erfindungsgemäße Verfahren (f) wird unter Verwendung eines Reduktionsmittels und gegebenenfalls eines Katalysators durchgeführt. Geeignete Systeme aus Reduktionsmitteln und Katalysatoren sind beispielsweise Wasserstoff in Kombination mit üblichen Hydrierungskatalysatoren, wie z.B. Raney-Nickel, Palladium oder Platin, ferner auch gegebenenfalls komplexe Metallhydride, wie z.B. Lithiumalanat, Natriumboranat und Natriumcyanoborhydrid, gegebenenfalls in Kombination mit sauren Katalysatoren, wie z.B

Salzsäure oder Essigsäure.

Verfahren (f) wird vorzugsweise in Gegenwart eines Verdünnungsmittels durchgeführt. Es können hierbei die gleichen Lösungsmittel verwendet werden, die oben für die zweite Stufe des erfindungsgemäßen Verfahrens (b) angegeben sind.

Die Reaktionstemperaturen können bei dem erfindungsgemäßen Verfahren (f) in einem größeren Bereich variiert werden. Im allgemeinen arbeitet man bei Temperaturen zwischen -20 °C und +100 °C, vorzugsweise bei Temperaturen zwischen 0 °C und +30 °C.

Die Reaktionsdurchführung, Aufarbeitung und Isolierung der Reaktionsprodukte erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Verbindungen der Formel (I) versickern extrem langsam im Boden; somit ist eine Belastung des Grundwassers praktisch ausgeschlossen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und

fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid(BUTACHLOR); 5-Amino-4-chlor-2-phenyl-2,3-dihydro-3-oxy-pyridazin (CHLORIDAZON); Ethyl-2-{[-(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETNYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy)-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy)-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitrophenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octyl-thiocarbonat (PYRIDATE); 2-[4-(6-Chlor-chinoxalin-2-yl oxy)-phenoxy]-propionsäure-ethylester (QUIZALOFOPETHYL); 2-[1-(Ethoxamino)-butylidenl-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino)-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

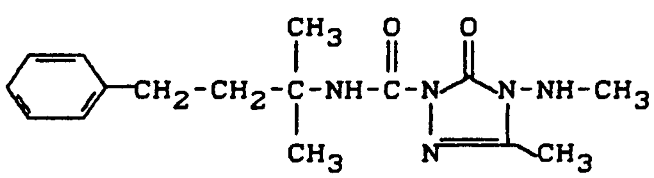

(Verfahren (a))

Eine Mischung aus 2,6 g (0,02 Mol) 4-Methylamino-5-methyl-2,4-dihydro-3H-1,2,4-triazol-3-on, 3,8 g (0,02 Mol) 1,1-Dimethyl-3-phenyl-propyl-isocyanat, 0,1 g Diazabicycloundecen (DBU) und 100 ml Acetonitril wird 12 Stunden bei 20 °C gerührt und anschließend im Wasserstrahlvakuum eingeengt. Der Rückstand wird in Methylenchlorid aufgenommen, mit Wasser gewaschen, mit Natriumsulfat getrocknet und filtriert. Das Filtrat wird eingeengt, der Rückstand durch Verreiben mit Diethylether/Petrolether zur Kristallisation gebracht und das kristalline Produkt durch Absaugen isoliert.

Man erhält 5,1 g (80% der Theorie) 2-(1,1-Dimethyl-3-phenyl-propylaminocarbonyl)-4-methylamino-5-methyl-2,4-dihydro-3H-1,2,4-triazol-3-on vom Schmelzpunkt 102 °C.

Analog Beispiel 1 und entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

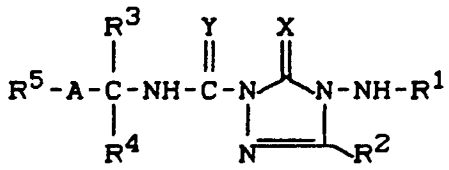

(I)

**Tabelle 2:** Herstellungsbeispiele für die Verbindungen der Formel (I)

| Bsp.-Nr. | A | $R^1$ | $R^2$ | $R^3$ | $R^4$ | $R^5$ | X | Y | Schmelzpunkt (°C) |
|---|---|---|---|---|---|---|---|---|---|
| 2 | $-CH_2-$ | $CH_3$ | $CH_3$ | $CH_3$ | $CH_3$ | 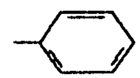 | O | O | 98 |
| 3 | $-CH_2-$ | $CH_3$ | 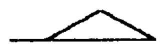 | $CH_3$ | $CH_3$ | 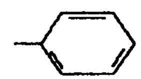 | O | O | 120 |
| 4 | $-CH_2CH_2-$ | $CH_3$ | 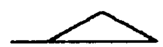 | $CH_3$ | $CH_3$ | 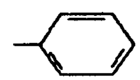 | O | O | 105 |
| 5 | $-CH_2CH_2-$ | $CH_3$ | $C_2H_5$ | $CH_3$ | $CH_3$ | 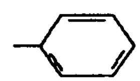 | O | O | 80 |
| 6 | $-CH_2-$ | $CH_3$ | $C_2H_5$ | $CH_3$ | $CH_3$ | 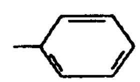 | O | O | 111 |
| 7 | $-CH_2-$ | $CH_3$ | 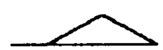 | H | $CH_3$ | 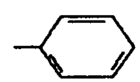 | O | O | 90 |
| 8 | $-C\equiv C-$ | $CH_3$ | $C_2H_5$ | $CH_3$ | $CH_3$ | 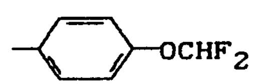 | O | O | (amorph) |

Tabelle 2 - Fortsetzung

| Bsp.-Nr. | A | $R^1$ | $R^2$ | $R^3$ | $R^4$ | $R^5$ | X | Y | Schmelzpunkt (°C) |
|---|---|---|---|---|---|---|---|---|---|
| 9 | $-CH_2CH_2-$ | $CH_3$ | $C_2H_5$ | $CH_3$ | $CH_3$ | 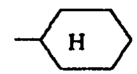 | O | O | 97 |
| 10 | $-CH_2CH_2-$ | $CH_3$ | $CH_3$ | $CH_3$ | $CH_3$ | 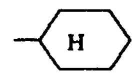 | O | O | 112 |
| 11 | $-CH_2CH_2-$ | $CH_3$ | 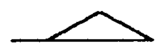 | $CH_3$ | $CH_3$ | 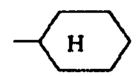 | O | O | 123 |
| 12 | $-(CH_2)_3-$ | $CH_3$ | 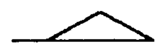 | $CH_3$ | $CH_3$ | 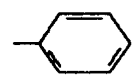 | O | O | 105 |
| 13 | $-CH_2CH_2-$ | $CH_3$ | 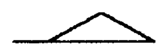 | $CH_3$ | $CH_3$ | 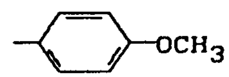 | O | O | 102 |
| 14 | $-CH_2-$ | $CH_3$ | $CH_3$ | $CH_3$ | $CH_3$ | 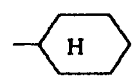 | O | O | 94 |
| 15 | $-C{\equiv}C-$ | $CH_3$ | $CH_3$ | $CH_3$ | $CH_3$ | 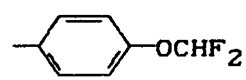 | O | O | 90 |

Tabelle 2 - Fortsetzung

| Bsp.-Nr. | A | $R^1$ | $R^2$ | $R^3$ | $R^4$ | $R^5$ | X | Y | Schmelzpunkt (°C) |
|---|---|---|---|---|---|---|---|---|---|
| 16 | -C≡C- | $CH_3$ | 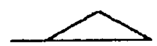 | $CH_3$ | $CH_3$ | 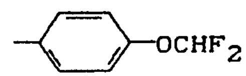 | O | O | 108 |
| 17 | $-(CH_2)_3-$ | $CH_3$ | $CH_3$ | $CH_3$ | $CH_3$ | 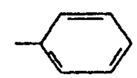 | O | O | 83 |

<u>Tabelle 2</u> - Fortsetzung

| Bsp.-Nr. | A | $R^1$ | $R^2$ | $R^3$ | $R^4$ | $R^5$ | X | Y | Schmelzpunkt (°C) |
|---|---|---|---|---|---|---|---|---|---|
| 18 | $-CH_2CH_2-$ | $CH_3$ | $CH_3$ | H | $CH_3$ | 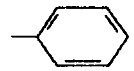 | O | O | 102 |
| 19 | $-CH_2CH_2-$ | $CH_3$ | $C_2H_5$ | H | $CH_3$ | 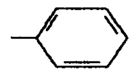 | O | O | 77 |
| 20 | $-CH_2CH_2-$ | $CH_3$ | 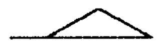 | H | $CH_3$ | 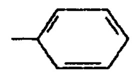 | O | O | 125 |
| 21 | $-CH_2CH_2-$ | $CH_3$ | $CH_3$ | $CH_3$ | $CH_3$ | 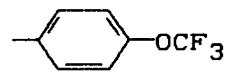 | O | O | 73 |
| 22 | $-CH_2CH_2-$ | $CH_3$ | 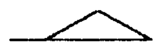 | $CH_3$ | $CH_3$ | 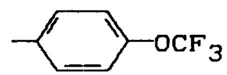 | O | O | 103 |
| 23 | $-CH_2CH_2-$ | $CH_3$ | $CH_3$ | $CH_3$ | $CH_3$ | 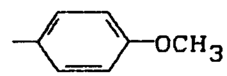 | O | O | (amorph) |
| 24 | $-CH_2CH_2-$ | $CH_3$ | $CH_3$ | H | $CH_3$ | 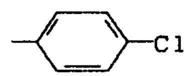 | O | O | 87 |

Tabelle 2 - Fortsetzung

| Bsp.-Nr. | A | $R^1$ | $R^2$ | $R^3$ | $R^4$ | $R^5$ | X | Y | Schmelzpunkt (°C) |
|---|---|---|---|---|---|---|---|---|---|
| 25 | $-CH_2CH_2-$ | $CH_3$ | $C_2H_5$ | H | $CH_3$ | 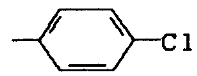 | O | O | 70 |
| 26 | $-CH_2CH_2-$ | $CH_3$ | 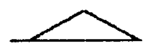 | H | $CH_3$ | 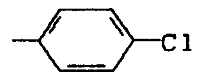 | O | O | (amorph) |
| 27 | $-CH_2CH_2-$ | $CH_3$ | $CH_3$ | H | $CH_3$ | 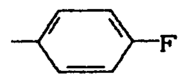 | O | O | (amorph) |
| 28 | $-CH_2CH_2-$ | $CH_3$ | $C_2H_5$ | H | $CH_3$ | 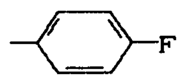 | O | O | 74 |
| 29 | $-CH_2CH_2-$ | $CH_3$ | 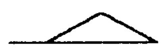 | H | $CH_3$ | 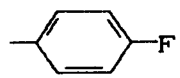 | O | O | (amorph) |
| 30 | $-CH_2CH-$ $CH_3$ | $CH_3$ | $CH_3$ | H | $CH_3$ | 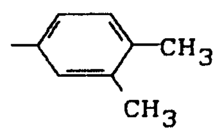 | O | O | 113 |
| 31 | $-CH_2CH-$ $CH_3$ | $CH_3$ | $C_2H_5$ | H | $CH_3$ | 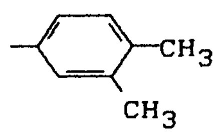 | O | O | 120 |

Ausgangsstoffe der Formel (II):

Beispiel (II-1)

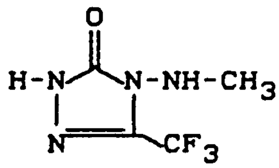

4,5 g (0,02 Mol) 4-Ethoxymethylenimino-5-trifluormethyl-2,4-dihydro-3H-1,2,4-triazol-3-on werden in 20 ml Tetrahydrofuran gelöst und unter Rühren bei 20 °C zu einer Suspension von 1,0 g (0,026 Mol) Lithiumaluminiumhydrid in 80 ml Tetrahydrofuran getropft. Nach Ende der Gasentwicklung läßt man auf Raumtemperatur kommen und rührt noch 12 Stunden bei dieser Temperatur (20 °C).

Unter Eiskühlung wird dann zunächst eine Mischung aus 25 ml Wasser und 25 ml Tetrahydrofuran zugetropft und schließlich mit 250 ml Wasser verdünnt. Nach Ansäuern mit 2N-Salzsäure wird mit Essigester ausgeschüttelt, die organische Phase mit Natriumsulfat getrocknet und filtriert. Vom Filtrat wird das Lösungsmittel im Wasserstrahlvakuum abdestilliert.

Man erhält 0,2 g (19 % der Theorie) 4-Methylamino-5-trifluormethyl-2,4-dihydro-3H-1,2,4-triazol-3-on als kristallinen Rückstand vom Schmelzpunkt 90 °C.

In entsprechender Weise erhält man die folgenden Ausgangsstoffe der allgemeinen Formel (II):

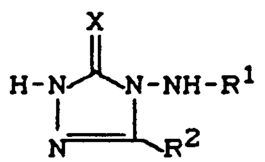

(II)

Tabelle 3

| Bsp.-Nr. | $R^2$ | $R^1$ | X | Schmelzpunkt °C |
|---|---|---|---|---|
| II-2 | $CH_3$ | $CH_3$ | O | 113 |
| II-3 | $n-C_3H_7$ | $CH_3$ | O | 76 |
| II-4 | $CH(CH_3)_2$ | $CH_3$ | O | 105 |
| II-5 | 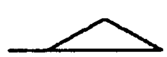 | $CH_3$ | O | 95 |
| II-6 | $C_2H_5$ | $CH_3$ | O | 101 |

Ausgangsstoffe der Formel (III):

Beispiel (III-1)

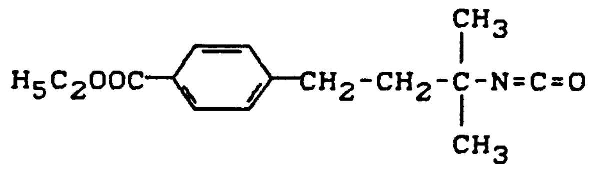

150 g (1,5 mol) Phosgen werden bei 15°C in 2,5 1 Chlorbenzol vorgelegt und in diese Lösung wird unter Rühren eine Lösung von 235 g (1,0 mol) 1,1-Dimethyl-3-(4-ethoxycarbonyl-phenyl)-propylamin in 1,0 1 Chlorbenzol tropfenweise eindosiert, wobei die Innentemperatur auf ca. 25°C ansteigt. Das Reaktionsgemisch wird dann zunächst auf ca. 70°C erwärmt und unter weiterem Einleiten von Phosgen (ca. 50 g/h) und unter Rühren langsam zum Rückfluß (ca. 127°C) erhitzt und nach 30 Minuten bei Rückfluß phosgeniert. Aus der klaren Lösung wird ca. 1 l Chlorbenzol zusammen mit überschüssigem Phosgen abdestilliert. Schließlich wird im Wasserstrahlvakuum eingeengt und der Rückstand im Ölpumpenvakuum destilliert.

Man erhält 253 g (97% der Theorie) 1,1-Dimethyl-3-(4-ethoxycarbonyl-phenyl)-propyl-isocyanat vom Siedebereich 125°C-130°C/0,1 mbar.

Analog Beispiel (III-1) können beispielsweise auch die in der nachstehenden Tabelle 4 aufgeführten Verbindungen der Formel (III) hergestellt werden:

$R^1$-N=C=X (III)

Tabelle 4: Beispiele für die Verbindungen der Formel (III)

| Bsp.-Nr. | $R^1$ | X | Physikalische Daten |
|---|---|---|---|
| III-2 | 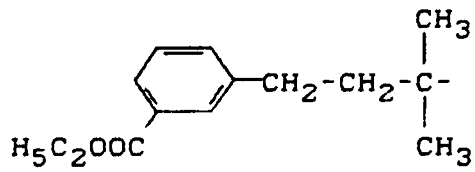 | O | |
| III-3 | 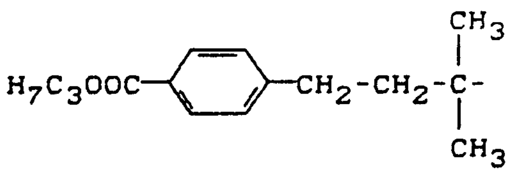 | O | Kp: 110-113° C bei 0,08 mbar |
| III-4 | 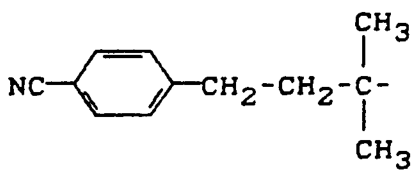 | O | Kp: 110° C bei 0,1 - 0,2 mbar |
| III-5 | 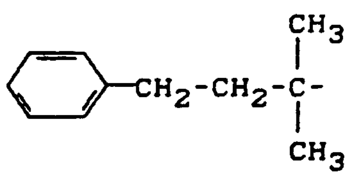 | O | |
| III-6 | 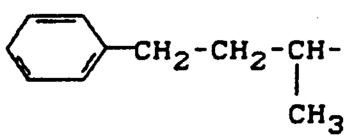 | O | |

Tabelle 4 - Fortsetzung

| Bsp.-Nr. | $R^1$ | X | Physikalische Daten |
|---|---|---|---|
| III-7 | 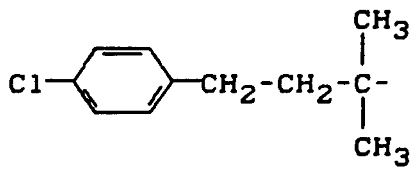 | O | Kp: 94°C bei 0,2 mbar |
| III-8 | 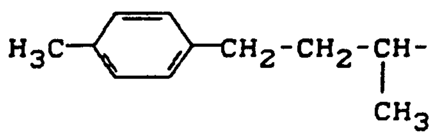 | O | |
| III-9 | 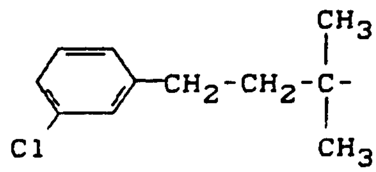 | O | |
| III-10 | 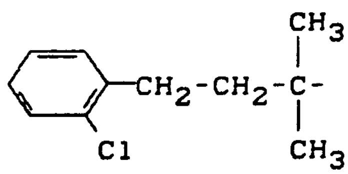 | O | |
| III-11 | 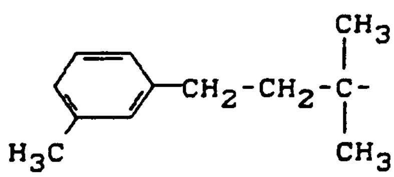 | O | |
| III-12 | 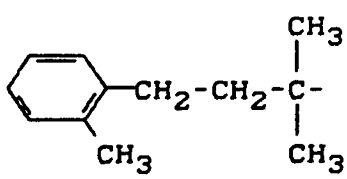 | O | |
| III-13 | 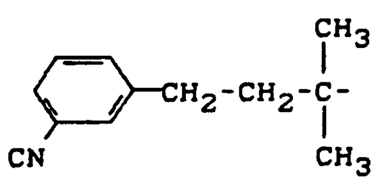 | O | |

Tabelle 4 - Fortsetzung

| Bsp.-Nr. | $R^1$ | X | Physikalische Daten |
|---|---|---|---|
| III-14 | 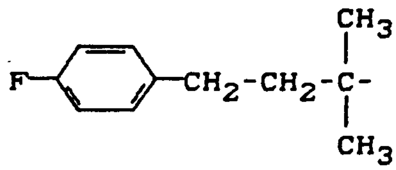 | O | |
| III-15 | 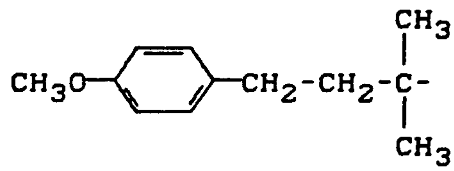 | O | |
| III-16 | 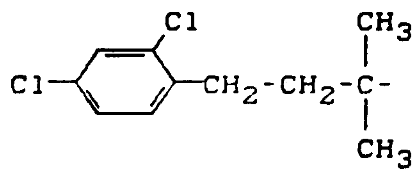 | O | |
| III-17 | 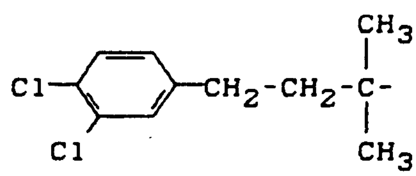 | O | |
| III-18 | 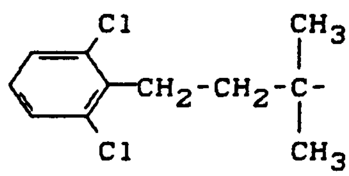 | O | |
| III-19 | 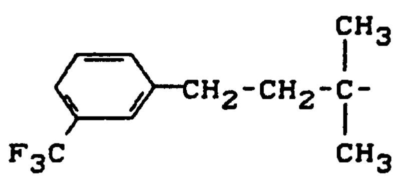 | O | |
| III-20 | 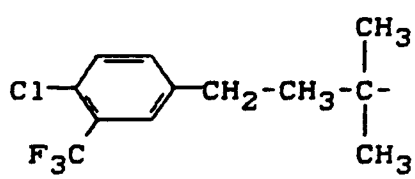 | O | |

Tabelle 4 - Fortsetzung

| Bsp.-Nr. | $R^1$ | X | Physikalische Daten |
|---|---|---|---|
| III-21 | 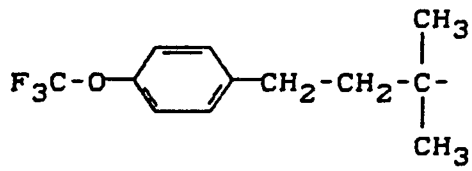 | O | |
| III-22 | 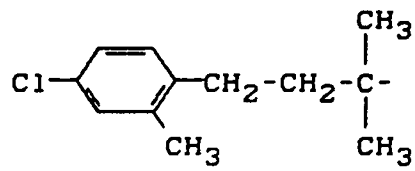 | O | |
| III-23 | 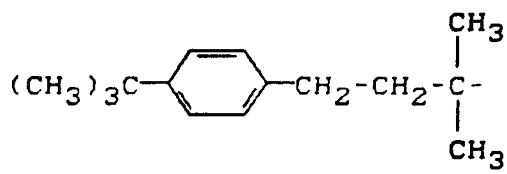 | O | |
| III-24 | 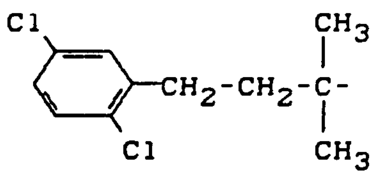 | O | |
| III-25 | 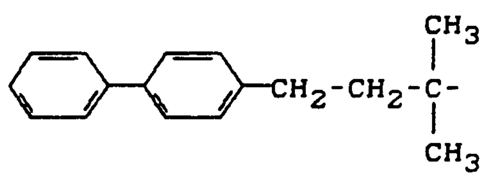 | O | |
| III-26 | 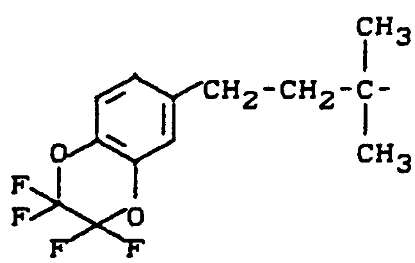 | O | |

Anwendungsbeispiele:

In den folgenden Anwendungsbeispielen wurde die nachstehend aufgeführte Verbindung als Vergleichssubstanz eingesetzt:

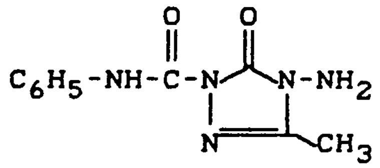 (A)

2-(Phenylaminocarbonyl)-4-amino-5-methyl-2,4-dihydro-3H-1,2,4-triazol-3-on (bekannt aus EP-A 294666, Beispiel 122, dort bezeichnet als "4-Amino-1-(N-phenylcarbamoyl)-3-methyl-triazolin-5-on".

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so, daß die jeweils gewünschten Wirkstoffmengen pro Flächeneinheit ausgebracht werden. Die Konzentration dar Spritzbrühe wird so gewählt, daß in 1 000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei der Bekämpfung von Unkräutern gegenüber der Vergleichssubstanz (A) zeigen in diesem Test z.B. die Verbindungen gemäß den Herstellungsbeispielen: (1), (3), (4), (5), (7) und (10).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei der Bekämpfung von Unkräutern gegenüber der Vergleichssubstanz (A) zeigen in diesem Test z.B. die Verbindungen gemäß den Herstellungsbeispielen: (1), (3) und (4).

**Patentansprüche**

1. Substituierte Triazolinone der allgemeinen Formel (I),

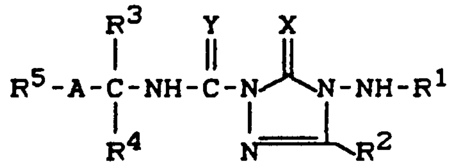

(I)

in welcher

| | |
|---|---|
| A | für Alkandiyl, Alkendiyl oder Alkindiyl steht, |
| $R^1$ | für Alkyl, Alkenyl, Alkinyl, Halogenalkyl, Halogenalkenyl, Halogenalkinyl, Cyanalkyl, Alkoxyalkyl, Cycloalkyl, Halogencycloalkyl, Alkylcycloalkyl oder Cycloalkylalkyl steht, |
| $R^2$ | für Alkyl, Halogenalkyl, Alkoxyalkyl oder Cycloalkyl steht, |
| $R^3$ | für Wasserstoff oder Alkyl steht, |
| $R^4$ | für Alkyl steht, |
| $R^5$ | für jeweils gegebenenfalls substituiertes Aryl oder Cycloalkyl steht und |
| X und Y | unabhängig voneinander für Sauerstoff oder Schwefel stehen, |

wobei folgende Verbindungen ausgenommen sind: 2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

**2.** Substituierte Triazolinone der allgemeinen Formel (I) gemäß Anspruch 1, dadurch gekennzeichnet, daß darin

| | |
|---|---|
| A | für jeweils geradkettiges oder verzweigtes Alkandiyl mit 1 bis 6 Kohlenstoffatomen, Alkendiyl mit 2 bis 6 Kohlenstoffatomen oder Alkindiyl mit 2 bis 6 Kohlenstoffatomen steht, |
| $R^1$ | für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Cyanalkyl oder Alkoxyalkyl mit 1 bis 6 Kohlenstoffatomen in den Alkyteilen, Cycloalkyl mit 3 bis 6 Kohlenstoffatomen, Halogencycloalkyl mit 3 bis 6 Kohlenstoffatomen und 1 bis 4 Halogenatomen, Alkylcycloalkyl oder Cycloalkylalkyl mit jeweils 1 bis 4 Kohlenstoffatomen im Alkylteil und 3 bis 6 Kohlenstoffatomen im Cycloalkylteil steht, |
| $R^2$ | für jeweils geradkettiges oder verzweigtes Alkyl mit 1 bis 8 Kohlenstoffatomen, Halogenalkyl mit 1 bis 8 Kohlenstoffatomen und 1 bis 17 gleichen oder verschiedenen Halogenatomen, Alkoxyalkyl mit 1 bis 6 Kohlenstoffatomen in den Alkylteilen oder Cycloalkyl mit 3 bis 6 Kohlenstoffatomen steht, |
| $R^3$ | für Wasserstoff oder für geradkettiges oder verzweigtes Alkyl mit 1 bis 4 Kohlenstoffatomen steht, |
| $R^4$ | für geradkettiges oder verzweigtes Alkyl mit 1 bis 4 Kohlenstoffatomen steht, |
| $R^5$ | für jeweils gegebenenfalls substituiertes Phenyl, Naphthyl oder Cyclohexyl steht, wobei für Phenyl als mögliche Substituenten vorzugsweise Halogen, Cyano, Nitro, Amino, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Phenyl, Phenoxy, Cyclohexyl oder gegebenenfalls durch Fluor und/oder Chlor bis zu vierfach substituiertes Alkylendioxy mit 1 oder 2 Kohlenstoffatomen ausgewählt sind und für Naphthyl oder Cyclohexyl als mögliche Substituenten geradkettige oder verzweigte Alkylgruppen mit 1 bis 4 Kohlenstoffatomen infrage kommen, und |
| X und Y | für Sauerstoff oder Schwefel stehen, |

wobei folgende Verbindungen ausgenommen sind: 2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

**3.** Substituierte Triazolinone der allgemeinen Formel (I) gemäß Anspruch 1, dadurch gekennzeichnet, daß darin

A für Methan-1,1-diyl (Methylen), Ethan-1,2-diyl (Ethylen, Dimethylen), Propan-1,2-diyl, Propan-1,3-diyl, Ethan-1,1-diyl, Propan-2,2-diyl, Ethen-1,2-diyl (Ethenylen, Vinylen) oder Ethin-1,2-diyl (Ethinylen) steht,

$R^1$ für Methyl, Ethyl, Propyl, Isopropyl, Butyl, Isobutyl, sec-Butyl oder tert-Butyl steht,

$R^2$ für Methyl, Ethyl, Propyl, Isopropyl, Butyl, Isobutyl, sec-Butyl, tert-Butyl, Fluormethyl, Chlormethyl, Difluormethyl, Dichlormethyl, Chlorfluormethyl, Trifluormethyl, Trichlormethyl, Chlordifluormethyl, Dichlorfluormethyl, Methoxymethyl, Methoxyethyl, Ethoxymethyl oder Cyclopropyl steht,

$R^3$ für Wasserstoff, Methyl, Ethyl, Propyl, Isopropyl, Butyl oder Isobutyl steht,

$R^4$ für Methyl, Ethyl, Propyl, Isopropyl, Butyl oder Isobutyl steht,

$R^5$ für gegebenenfalls durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Difluormethyl, Trifluormethyl, Difluormethoxy, Trifluormethoxy, Difluormethylthio, Trifluormethylthio, Trifluormethylsulfinyl, Trifluormethylsulfonyl, Methylsulfinyl, Methylsulfonyl, Cyclohexyl, Phenyl oder Phenoxy substituiertes Phenyl oder für gegebenenfalls durch Methyl, Ethyl, Propyl, Isopropyl, Butyl, Isobutyl, sec-Butyl oder tert-Butyl substituiertes Cyclohexyl steht,

X für Sauerstoff oder Schwefel steht und

Y für Sauerstoff oder Schwefel steht, wobei folgende Verbindungen ausgenommen sind:

2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

**4.** Substituierte Triazolinone der allgemeinen Formel (I) gemäß Anspruch 1, dadurch gekennzeichnet, daß darin

A für Methan-1,1-diyl, Ethan-1,2-diyl, Propan-1,3-diyl oder Ethin-1,2-diyl steht,

$R^1$ für Methyl oder Ethyl steht,

$R^2$ für Methyl, Ethyl, Propyl, Isopropyl oder Cyclopropyl steht,

$R^3$ für Wasserstoff, Methyl oder Ethyl steht,

$R^4$ für Methyl oder Ethyl steht,

$R^5$ für gegebenenfalls durch Fluor, Chlor, Brom, Methyl, Ethyl, Methoxy, Ethoxy, Trifluormethyl, Difluormethoxy, Trifluormethoxy, Methylthio, Methylsulfinyl, Methylsulfonyl, Trifluormethylthio, Trifluormethylsulfinyl oder Trifluormethylsulfonyl substituiertes Phenyl oder für Cyclohexyl steht,

X für Sauerstoff steht und

Y für Sauerstoff oder Schwefel steht,

wobei folgende Verbindungen ausgenommen sind:

2-((1,1-Dimethyl-2-phenyl-ethyl)-amino-carbonyl)-4-methylamino-5-methyl-2,4-dihydro-3H-1,2,4-triazol-3-on, 2-((1-Methyl-2-(3,4-dimethoxy-phenyl)-ethyl)-amino-carbonyl)-4-ethylamino-5-methyl-2,4-dihydro-3H-1,2,4-triazol-3-on und 2-((1-Methyl-3,3-dimethyl-3-phenyl-propyl)-amino-carbonyl)-4-methylamino-5-methyl-2,4-dihydro-3H-1,2,4-triazol-3-on.

**5.** Verfahren zur Herstellung von substituierten Triazolinonender allgemeinen Formel (I) gemäß Anspruch 1, dadurch gekennzeichnet, daß man

(a) Triazolinone der allgemeinen Formel (II)

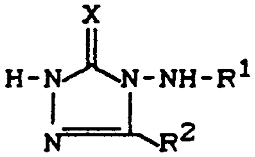

(II)

in welcher

$R^1$, $R^2$ und X die in Anspruch 1 angegebene Bedeutung haben,

mit Iso(thio)cyanaten der allgemeinen Formel (III),

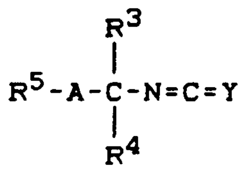

(III)

in welcher

A, $R^3$, $R^4$, $R^5$ und Y die in Anspruch 1 angegebene Bedeutung haben,

gegebenenfalls in Gegenwart eines Verdünnungsmittels und gegebenenfalls in Gegenwart eines Reaktionshilfsmittels umsetzt, oder daß man

(b) Triazolinone der allgemeinen Formel (IV),

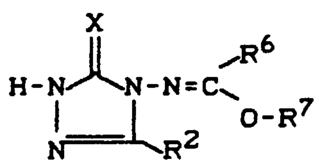

(IV)

in welcher

$R^2$ und X die in Anspruch 1 angegebene Bedeutung haben und

$R^6$ und $R^7$ für Alkyl, Alkenyl, Alkinyl oder Cycloalkyl stehen, sowie $R^6$ auch für Wasserstoff steht,

mit Iso(thio)cyanaten der allgemeinen Formel (III),

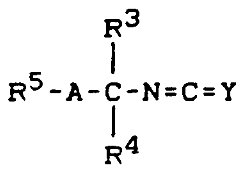

(III)

in welcher

A, $R^3$, $R^4$, $R^5$ und Y die in Anspruch 1 angegebene Bedeutung haben,

gegebenenfalls in Gegenwart eines Verdünnungsmittels und gegebenenfalls in Gegenwart eines Reaktionshilfsmittels umsetzt und die so erhaltenen Verbindungen der allgemeinen Formel (V),

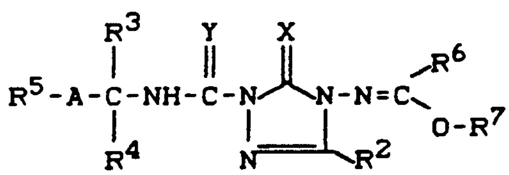

(V)

in welcher

A, $R^2$, $R^3$, $R^4$, $R^5$, $R^6$, $R^7$, X und Y die in Anspruch 1 angegebene Bedeutung haben,

in einem zweiten Reaktionsschritt mit einem Reduktionsmittel der Formel (VI) oder (VII),

$MBH_4$ (VI)

$MAlH_4$ (VII)

worin

M für ein Alkalimetallatom steht,

gegebenenfalls in Gegenwart eines Verdünnungsmittels zu den Verbindungen der allgemeinen Formel (VIII),

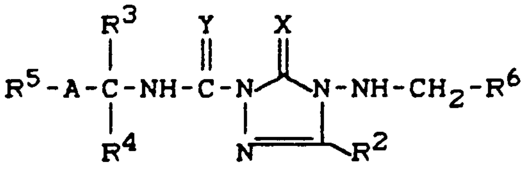

(VIII)

in welcher

A, $R^2$, $R^3$, $R^4$, $R^5$, $R^6$, X und Y die oben angegebene Bedeutung haben,

umsetzt, oder daß man

(c) Aminotriazolinone der allgemeinen Formel (IX)

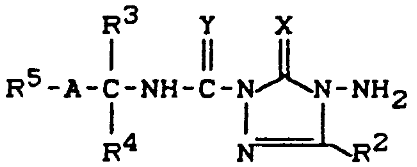

(IX)

in welcher

A, $R^2$, $R^3$, $R^4$, $R^5$, X und Y die oben angegebene Bedeutung haben,

mit Orthocarbonsäureestern der allgemeinen Formel (X)

$R^6$-C($OR^7$)$_3$ (X)

in welcher

$R^6$ und $R^7$ die oben angegebene Bedeutung haben,

gegebenenfalls in Gegenwart eines Verdünnungsmittels und gegebenenfalls in Gegenwart eines Reaktionshilfsmittels zu Verbindungen der allgemeinen Formel (V)

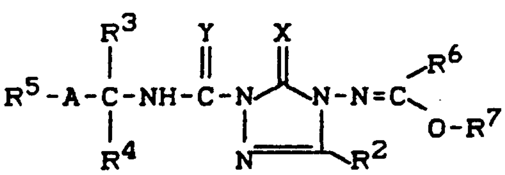

(V)

in welcher

A, $R^2$, $R^3$, $R^4$, $R^5$, $R^6$, $R^7$, X und Y die oben angegebene Bedeutung haben,

umsetzt und diese Verbindungen der Formel (V) in einem zweiten Reaktionsschritt mit einem Reduktionsmittel der Formel (VI) oder (VII)

$MBH_4$ (VI)

$MAlH_4$ (VII)

worin

M für ein Alkalimetallatom steht,

gegebenenfalls in Gegenwart eines Verdünnungsmittels zu den Verbindungen der allgemeinen Formel (VIII)

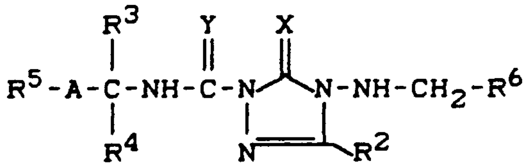

(VIII)

in welcher

A, $R^2$, $R^3$, $R^4$, $R^5$, $R^6$, X und Y die oben angegebene Bedeutung haben,

umsetzt, oder daß man

(d) Triazolinone der allgemeinen Formel (II)

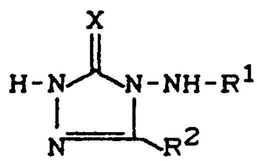

(II)

in welcher

$R^1$, $R^2$ und X die oben angegebene Bedeutung haben,

mit Chlor(thio)kohlensäurederivaten der allgemeinen Formel (XI)

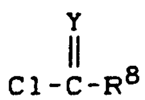

(XI)

in welcher

Y die oben angegebene Bedeutung hat und

$R^8$ für Alkoxy, Aryloxy, Aralkyloxy oder Halogen steht,

gegebenenfalls in Gegenwart eines Verdünnungsmittels und gegebenenfalls in Gegenwart eines Reaktionshilfsmittels umsetzt und die so erhältlichen Verbindungen der allgemeinen Formel (XII)

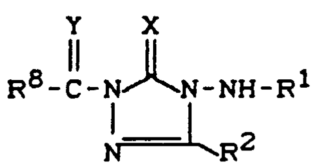

(XII)

in welcher

$R^1$, $R^2$, $R^8$, X und Y die oben angegebene Bedeutung haben,

in einer anschließenden zweiten Stufe mit Aminen der allgemeinen Formel (XIII)

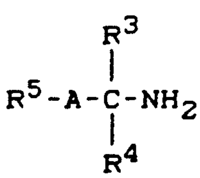

(XIII)

in welcher

A, $R^3$, $R^4$ und $R^5$ die oben angegebene Bedeutung haben,

gegebenenfalls in Gegenwart eines Verdünnungsmittels und gegebenenfalls in Gegenwart eines Reaktionshilfsmittels zu Verbindungen der Formel (I) umsetzt, oder daß man

(e) Triazolinone der allgemeinen Formel (II)

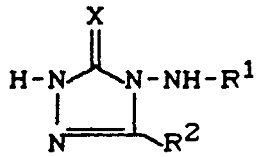

(II)

in welcher

$R^1$, $R^2$ und X die oben angegebene Bedeutung haben,

mit (Thio)Carbamidsäurederivaten der allgemeinen Formel (XIV)

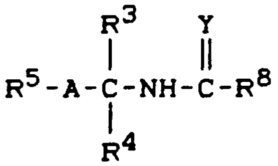

(XIV)

in welcher

A, $R^3$, $R^4$, $R^5$, $R^8$ und Y die oben angegebene Bedeutung haben,

gegebenenfalls in Gegenwart eines Verdünnungsmittels und gegebenenfalls in Gegenwart eines Reaktionshilfsmittels umsetzt, oder daß man

(f) Triazolinon-Hydrazone der allgemeinen Formel (XV)

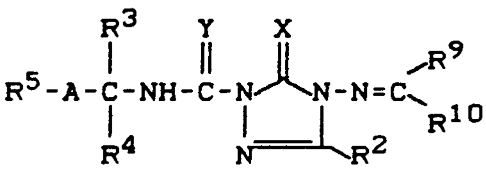

(XV)

in welcher

A, $R^2$, $R^3$, $R^4$, $R^5$, X und Y die oben angegebene Bedeutung haben und

$R^9$ und $R^{10}$ für Wasserstoff, Alkyl, Alkenyl, Alkinyl oder Cycloalkyl stehen,

mit Reduktionsmitteln, gegebenenfalls in Gegenwart von Katalysatoren und gegebenenfalls in Gegenwart von Verdünnungsmitteln umsetzt.

**6.** Herbizide Mittel, gekennzeichnet durch einen Gehalt an mindestens einem substituierten Triazolinon der allgemeinen Formel (I) gemäß Anspruch 1.

**7.** Verfahren zur Bekämpfung von Unkraut, dadurch gekennzeichnet, daß man ein substituiertes Triazolinon der Formel (I) gemäß Anspruch 1 auf die Unkräuter oder ihren Lebensraum einwirken läßt.

**8.** Verwendung von substituierten Triazolinonen der Formel (I) gemäß Anspruch 1 zur Bekämpfung von unerwünschtem Pflanzenwachstum.

**9.** Verfahren zur Herstellung von herbiziden Mitteln, dadurch gekennzeichnet, daß man substituierte Triazolinone der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 92 10 4042

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | EP-A-0 399 294 (BAYER AG)<br>* das ganze Dokument, insbesondere Seite 14, erste Verbindung, Seite 15, erste Verbindung und Seite 16, letzte Verbindung *<br>----- | 1-9 | C07D249/12<br>A01N47/38 |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 29 JUNI 1992 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)